# EUROPEAN PATENT APPLICATION

(11) **EP 4 529 831 A1**
(43) Date of publication of application: **02.04.2025**
(21) Application number: 24203652.3
(22) Date of filing: 30.09.2024
(51) Int. Cl.: A61B 5/00, A61B 5/145

(54) **ANALYTE SENSOR APPLICATOR**

(30) Priority: 29.09.2023 GB 202315019
(71) Applicant: Glucorx Technologies Limited, Dingwall IV15 9QF (GB)
(72) Inventor: MCCULLOCH, Andrew, Framlingham, IP13 9EZ (GB); MURRAY-SCOTT, Cerys Rohann, Dingwall, IV15 9QF (GB)
(74) Representative: Marks & Clerk LLP

(57) **Abstract**

An inserter device for securing a sensor module to the skin of a user, and a kit comprising the inserter device and a sensor module. The inserter device is configured, during use, to drive a pointed end of the sensor module at least partially into the skin of the user. The inserter device comprises a body housing a carriage for receiving the sensor module therein before use, and a collar radially surrounding the carriage and movable relative to the body in an axial direction from a first position to a second position. The collar comprises one or more engagement members to configured to engage one or more corresponding abutment surfaces on an inner surface of the body in the first position and thereby prevent relative movement of the collar and the body. The one or more engagement members and/or abutment surfaces are configured such that an axial force applied to the body causes the one or more engagement members and abutment surfaces to disengage from one another, thereby facilitating movement of the collar from the first position to the second position.

## Description

### Field of Invention

The present disclosure relates to a device and method for securing a sensor module to the skin of a user, and in particular but not exclusively to an applicator for securing an analyte sensor to a user.

### Background

Traditionally, monitoring blood glucose levels of a patient involved a process where a finger prick blood test obtained a small drop of blood that was placed on a test strip that inserted into a glucometer. The glucometer read the strip and provided a digital reading of the individual's blood sugar level.

Recently, finger prick blood tests have been replaced by insertable (implantable), in vivo, analyte sensors that are inserted into the skin of the patient where they remain at all times, enabling substantially continuous measurements to be taken, which is advantageous compared to finger prick tests that provide only snapshot readings at a small number of times a day. These types of implantable analyte sensors are typically coupled to a sensor module having a housing placed on the surface of the patient's skin. For example, the rearwardly protruding part of the analyte sensor is inserted into the skin-facing surface of the housing the sensor module, where it is coupled inside the sensor module to control electronics. This ensures only the sensor module itself is visibly exposed to the outside environment and the entry point of the insertable analyte sensor in the skin is at least partially protected underneath the sensor module. The sensor module control electronics process measurement signals from the inserted sensor and transmit any relevant information to, for example, the patient's smartphone or other mobile device. These types of systems are sometimes known as continuous analyte monitoring systems.

Typically, continuous analyte monitoring systems require the analyte sensor to be replaced at predetermined intervals and this may require the patient to insert the analyte sensor themselves in an unsupervised environment. In order to simplify this process, known analyte monitoring systems are provided with an inserter device which applies a predetermined amount of force to the insertable analyte sensor to safely insert it into the patient's skin, and at the same time to position any accompanying sensor module at the surface of the patient's skin. EP2393417B1 proposes a continuous analyte monitoring system.

In known continuous analyte monitoring systems, such as that of EP2393417B1, the sensor module is initially inside of the inserter device. An adhesive pad or patch (sometimes referred to as an epidermal support patch) is provided on the skin-facing end of the inserter device. During use, the inserter device inserts the analyte sensor into the skin, places the sensor module onto the adhesive pad which is thereby secured to the patient's skin. The force of the sensor module and skin-facing rim of the inserter device during activation ensures the entire area of the adhesive pad is pressed securely onto the skin.

In order to ensure that the sensor module is positioned in a clinically relevant position (i.e. for measuring analytes in interstitial fluid), an analyte sensor is preferably attached to a user's skin with as little lateral motion as possible. An ideal movement of the analyte sensor during delivery would be completely normal to the user's skin, so that the needle of the sensor enters the body tissue smoothly and the sensor tail does not shift or buckle, while the ideal retraction of the needle would similarly be such that dragging forces on the sensor tail do not move it out of position. In existing insertion devices, angular deviation from this normal movement direction is typically reduced via complex spring loaded mechanisms, requiring large numbers of component pieces. As a result, these designs are both relatively costly and difficult to manufacture, and have a large number of potential failure points that may prevent or impact the positioning of the sensor module.

The applicant has therefore recognised that an improved applicator or inserter device for a sensor module is required.

### Summary

In general terms, the present disclosure is directed to an applicator or insertion device for securing a sensor module to the skin of a patient. Unlike in known systems, which comprise complex spring loaded mechanisms to control a movement direction of the sensor module during delivery processes, the inserter device of the present disclosure provides a design with a reduced number of components, providing the same or superior delivery performances to prior art devices while reducing the number of potential failure points and a cost/complexity of manufacture.

As a result of the above described features, the inserter device, analyte sensor, and sensor module of the present disclosures may provide a more reliable and cost effective means for a user to e.g. monitor blood glucose levels.

Thus, according to a first aspect of the disclosure, there is provided an inserter device for securing a sensor module to the skin of a user, the inserter device configured, during use, to drive a pointed end of the sensor module at least partially into the skin of the user, the inserter device comprising:
a body housing a carriage for receiving the sensor module therein before use;
a collar radially surrounding the carriage and movable relative to the body in an axial direction from a first position to a second position, the collar comprising one or more engagement members to configured to engage one or more corresponding abutment surfaces on an inner surface of the body in the first position and thereby prevent relative movement of the collar and the body;
wherein the one or more engagement members and/or abutment surfaces are configured such that an axial force applied to the body causes the one or more engagement members and abutment surfaces to disengage from one another, thereby facilitating movement of the collar from the first position to the second position.

The inserter device may be configured such that, in use, the sensor module is secured to the skin of the user when the collar is in the second position. Advantageously therefore, the engagement of the engagement members and the abutment surfaces prevents the movement of the collar to the second position until a (high) force is applied to the body, thereby ensuring that the user is applying a force to the inserter device that is sufficient to secure the sensor module to the user's skin.

Optionally therefore, the collar is configured to move in the axial direction relative to the body upon the application of the axial force that causes the one or more one or more engagement members and abutment surfaces to disengage from one another.

In implementations, at least one of (i) the one or more engagement members and (ii) the abutment surfaces comprises a deformable section, wherein the axial force deforms the deformable section thereby disengaging the one or more engagement members and abutment surfaces from one another. The deformable sections may be elastically deformable.

Optionally, the one or more engagement members comprise the deformable sections; and the abutment members are configured such that the axial force causes the abutment members to deform the deformable sections radially inwards.

Optionally, the carriage comprises a plurality of gripping members configured to hold or secure the sensor module within the carriage, the gripping members configured to release the sensor module upon movement of the collar to the second position.

Advantageously, during use the gripping members may be configured to release the sensor module when the collar is in the second position. In implementations, the gripping members may be deformable by a normal force exerted on the carriage by the user's skin, the deformation causing the gripping members to release the sensor module.

Additionally or alternatively, the collar may comprise a plurality of jaw posts, the jaw posts each configured to engage a respective one of the gripping members in the first position and exert a radially inward force on the gripping members. The jaw posts and gripping members may be configured to disengage due to or upon movement of the collar to the second position. The jaw posts may secure the gripping members in a position for holding the sensor module, while the gripping members themselves may be freely movable between a sensor holding position and a sensor release position, or otherwise biased to the sensor release position.

Advantageously therefore, the provision of the collar jaw posts may facilitate the automatic release of the sensor module when the collar is in the second position, while securely retaining the sensor module when the collar is in the first position.

Optionally, the inserter device comprises a force applicator, the force applicator configured to apply a second axial force from the body to the collar. The force applicator may be e.g. a spring or other suitable force application mechanism. The force applicator may be configured increase or enhance or otherwise alter an axial force applied by the user. Additionally or alternatively, the spring may be configured to bias the collar to the first position.

Optionally, the carriage comprises a protrusion configured, during use, to guide the at least one pointed end of the sensor module to pierce the skin of the user when the collar is in the second position. The protrusion may be a cannula, needle, or other element suitable for guiding the pointed end of the sensor module.

Optionally, the inserter device comprises an automatic cannula retraction mechanism. The force applicator may be configured to operate as the automatic cannula retraction mechanism. For example, a spring may be configured to bias the collar to the first position, thereby retracting the cannula upon the removal or reduction of an axial force from the user.

Advantageously, biasing the collar to the first position and thereby automatically extracting the cannula from the user's skin may reduce a risk of accidental injury to the user, e.g. after securing the sensor module to the user.

Optionally, the inserter device comprises a cap removably connected to the body, the cap configured to enclose a cavity of the body, the cavity for housing the sensor module. Further optionally, the cap and/or body may comprise one or more protrusions and/or grooves configured to engage with one another, the protrusions and grooves configured such that the removal of the cap requires both axial and rotational movements relative to the body.

The cap may comprise a sensor module activation member, wherein the cap is configured such that the process of removing the cap from the body causes the sensor module activation member to activate the sensor module housed within the body.

Advantageously, the inserter device may therefore be configured such that the removal of the cap activates the sensor module and primes the device for use in a single step or sequence, simplifying the use of the inserter device for the user.

Optionally, the inserter device comprises a seal ring removably positioned between the body and the cap, the seal ring configured to prevent the activation of the sensor module by the sensor module activation member. The seal ring may comprise or be formed from an elastically deformable or elastomeric material, as aid the user in its removal.

Advantageously, the seal ring may restrict a relative axial movement of the body and the cap prior to its removal from the inserter device, thereby preventing or restricting an accidental removal of the cap or activation of the sensor module by the sensor module activation member.

Optionally, a first end of the cap comprises a first flange, the first end of the cap being axially proximate to the body; the body comprises a second flange; and the inserter device comprises a gasket positioned between the first flange and the second flange. The first flange may further comprise an engagement surface configured to engage with the gasket.

Advantageously, gasket may restrict an initial movement of the cap relative to the body, thereby positionally securing the cap and the body and preventing or restricting an accidental removal of the cap or activation of the sensor module by the sensor module activation member.

Optionally, the inserter device comprises a second cavity between the body and the first flange, the cavity configured to receive the gasket.

Advantageously, by receiving the gasket within a cavity during the cap removal process, the gasket may be provided without hindering, restricting or opposing movement of the cap during the cap removal process.

Optionally, the cap further comprises one or more apertures for insertion of a gas into the cavity. The gas may be a sterilising gas, such as Ethylene Oxide, to thereby provide a sterile environment for the sensor module and enhance a shelf life of the inserter device.

According to a second aspect of the disclosure there is provided kit of parts for securing a sensor module to the skin of a user, the kit of parts comprising:
an inserter device according to the first aspect of the disclosure; and
a sensor module, the sensor module having a non-biocompatible adhesive on a surface thereof for securing the sensor module to the user;
the inserter device configured to, during use, move the sensor module into contact with the user to thereby secure the sensor module to user using said non-biocompatible adhesive.

Optionally, the kit comprises an epidermal support patch having a biocompatible adhesive on a surface thereof for securing the patch to the skin of a user; and
wherein securing the sensor module to the user comprises affixing the patch to the user and moving the sensor module into contact with the patch, to thereby secure the sensor module to patch using said non-biocompatible adhesive.

The advantages and various implementations described above in connection with the corresponding features of the first aspect also apply to the second aspect of the disclosure.

### Brief Description of the Drawings

These and other aspects will now be described, by way of example only, with reference to the accompanying figures in which:
Figure 1 illustratively shows an exploded view of an inserter device according to the present disclosure.
Figure 2 illustratively shows a cross sectional view of the assembled inserter device of Figure 1.
Figure 3 illustratively shows an assembled view of the inserter device of Figure 1.
Figures 4a-c illustratively show the inserter device of Figure 1 in use.
Figures 5a-d illustratively show cross sectional views of the inserter device of Figure 1 in use.
Figures 6a-d illustratively show further views of an example inserter device according to the present disclosure in use.
Figures 7a-c illustratively show an inserter device and sensor module according to the present disclosure.
Figures 8a-d illustratively show steps for removing a cap of an inserter device according to the present disclosure.
Figures 9a-d illustratively show cross sectional view of steps for removing a cap of an inserter device according to the present disclosure.
Figure 10 illustratively shows an epidermal support patch according to the present disclosure.
Figures 11a-d illustratively show various views of a further inserter device according to the present disclosure.
Figures 12a-c illustratively show various views of a further inserter device according to the present disclosure.
Figures 13a and b illustratively show cross section views of the inserter device according to Figures 12a-c.
Figures 14a-c illustratively show various views of a further inserter device according to the present disclosure.

### Detailed Description

The present disclosure generally relates to an applicator or inserter device for sensor module comprising a sensing device such as an analyte sensor. The inserter device comprises an assembly configured to allow the user to attach the sensor module to an area of their body, for example the back of the upper arm or their abdomen. The inserter device is configured to securely hold the sensor module within a sterile boundary for the shelf-life of the product (or until use). To operate the inserter device, the user removes a cap from the assembly and pushes the device onto the skin. Through this process, the sensor module is pushed onto the body, and may be held in place with e.g. a pre-exposed adhesive patch attached on the underside of the device or on the target area of the user's skin. Simultaneously, a slotted needle or cannula in the inserter device pierces the skin, allowing the sensing device of the sensor module to be implanted into the epidermis. As the inserter device is pulled off and away from the user's skin, the slotted needle is removed from the skin along with it, leaving the sensing device embedded in the body.

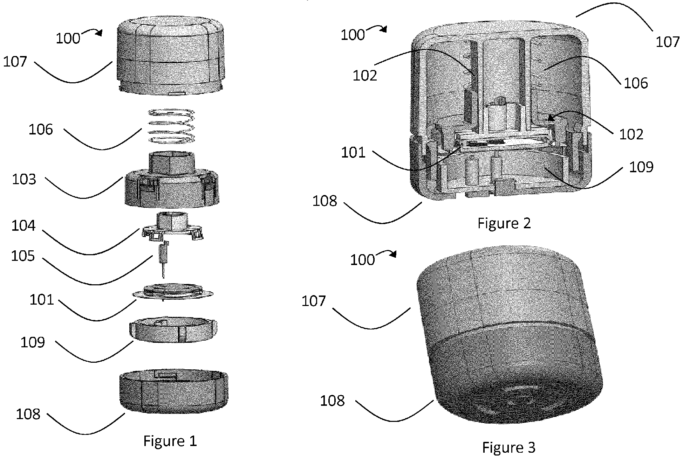

Figure 1 illustratively shows an exploded view of an applicator or inserter device 100 according to the present disclosure. The inserter device 103 comprises a body 107 and a removable cap 108 covering a forward end thereof (the term forward referring to the direction that faces the user's skin during use as opposed to rearward which refers to the opposite direction). The term 'axial' or 'axial direction' is used herein in reference to the direction of separation between the forward and rearwards ends, while 'radial' or 'radial direction' is used to refer to a direction or plane perpendicular to the axial direction.

Inside the space or cavity enclosed by the body 107 and removable cap 108, the inserter device 103 comprises a carriage, grip or holder 104 for receiving the sensor module 101 therein before use. The carriage 104 is configured to release the sensing module 101 upon contact with the skin of the user.

The carriage 104 is also provided with a cannula 105, needle or other corresponding sharp configured to hold and/or guide the analyte sensor of the sensor module 101 and pierce the skin as the carriage 104 with sensor module 101 is driven forwards to implant or insert the sensor into the user's skin.

The space enclosed by the body 107 further comprises a collar 103 configured to move relative to the body 107. The collar 103 surrounds the carriage 104 prior to and post use of the insertion device, thereby protecting both the sensor module 101 from damage and protecting the user from accidental injury, e.g. from the cannula 105.

The inserter device 100 may further comprise a force applicator such as a spring 106 or other force generating mechanism, coupled between the body 107 and the collar 103. The spring 106 may exert a force on the collar 103 to bias the collar to a first or exposed position when not in use. The spring 106 or other force generator may be provided about the body 107 and the collar 103, e.g. about a central rail or pillar 102 of the body 107 that is connected to the carriage 104 via an opening in the collar 103. Alternatively, the collar 103 may be maintained in position by e.g. gravity and one or more stops on central rail 102 or an internal wall of the body 107. The central rail 102 may guide the relative (axial) movement of the body 107 and collar 103, thereby reducing deviations in the angle of the force exerted by the user from the ideal (i.e. normal to the skin) direction.

The cap 108 comprises a start-up ring or activator 109 configured to start or activate the sensor module during the removal of the cap 108. Start-up ring 109 enables the sensor module to be turned-ON immediately prior to use in the same step as removing the cap 108 from the body 107, thereby simplifying the use of the inserter device 100 for the user and extending a shelf life of the device by e.g. reducing a battery drain of the sensing device prior to use.

Figures 2 and 3 illustratively show views of an assembled inserter device 100 comprising a sensing module 101 prior to removal of the cap 107.

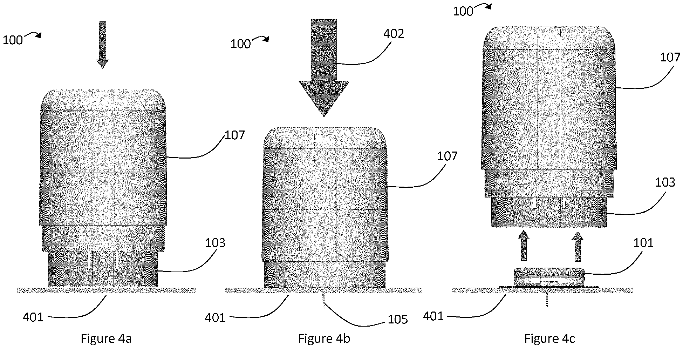

Figures 4a-c depict example steps for using an inserter device such as inserter device 100 to secure a sensing module to the skin of a user. In Figure 4a, and after removing a cap 108 from the inserter device 100, the user brings the inserter device 100 into contact with the target area of their skin 401. In Figure 4a, the collar is in a first position, also called an extended position. In Figure 4b, the user exerts an axial force 402 on the inserter device 100 or otherwise presses the body 107 of the device towards their skin 401 until the collar 103 moves inside the body 105. The movement of the body 107 towards their skin causes the cannula 105 to pierce the user's skin 401, thereby inserting the sensor device 101 into the epidermis. In Figure 4b, the collar is shown in a second position, also called a retracted position. In Figure 4c, the user removes the inserter device 100 from their body, retracting cannula 105 from their skin 105 and leaving being the sensor device 101. The collar 103 returns to its original (i.e. first) position, being at least partially exposed from an opening of the body 107 thereby radially covering or surrounding the cannula 105 and preventing accidental injury to the user. The sensor device 101 may be secured to the skin with an adhesive patch that is either placed on a target area of the skin, pre-attached to the sensor device itself, or attached to the inserter device 101.

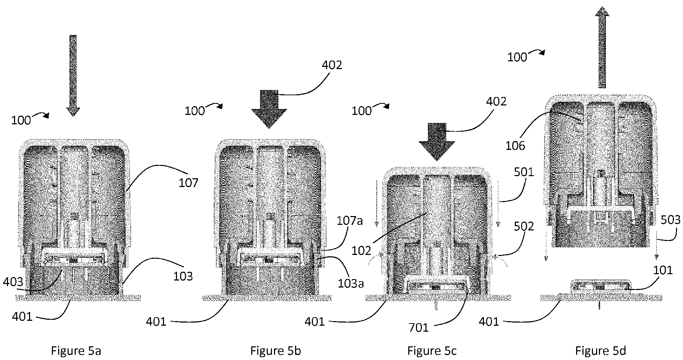

Figures 5a-d illustrate cross sectional views of the inserter device 100 during the steps depicted in Figures 4a-c. As in Figure 4a, in Figure 5a the user brings the inserter device 100 into contact with the target area of their skin 401. The collar 103 may comprise one or more guide slits 403 configured to receive corresponding guide protrusions (not shown) of the body. The guide slits and protrusions may limit a maximum allowed axial movement of the collar 103 relative to the body 107. Additionally or alternatively, the guide slits 403 may be used in combination with the cap 108, as described further below.

In Figure 5b, the user begins exerting a force 402 on the body of the device. Relative movement of the body 107 and collar 103 is resisted by corresponding engagement members, engagement surfaces, hooks and/or other protrusions 103a of the collar 103. The protrusions 103a engage corresponding slots, hooks, recesses, cam surfaces, abutment surfaces and/or other protrusions 107a of the body 107, thereby preventing insertion of the sensor module into the skin when an insufficient force is applied by the user. In the depicted implementation, the collar protrusion 103a is provided on a deformable wall section of the collar 103, however it will be understood that either or both of protrusions 107a and 103a may be deformable or elastically deformable, or otherwise provided on a deformable or an elastically deformable wall section of the body 107 or collar 103, respectively.

In Figure 5c, force 402 is sufficient to cause the body protrusion 107a to push the deformable wall section(s) of collar 103 (and therefore collar protrusions 103a) radially inwards 502, thereby allowing relative movement 501 of the body 107 and collar 103. The release of the body 107 (i.e. resulting from the user applying a sufficiently high axial or downward force to the body 107) results in movement of the body 107 towards the user's skin 401. Carriage 104 is connected or attached to body 107 via the central rail 102, and as such the axial or downward movement of the body 107 results in corresponding downward movement of the carriage 104. Thus, the cannula 105 pierces the user's skin 401 and inserts the sensor device 101 into the epidermis, as shown in Figure 4b.

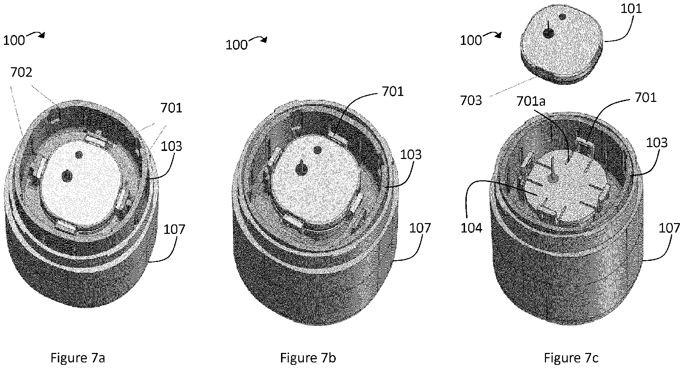

Carriage 104 is further configured such that the collar moving to the second position and/or contact with the user's skin 401 results in the release of the sensor module 101 from the carriage 104. In the depicted implementation, carriage 104 comprises one or more grip jaws or holding arms 701 for holding the sensor module 101 within the carriage 104. Grip jaws 701 are shown in more detail in e.g. Figures 7a-c. In Figure 5c, the grip jaws 701 move radially outwards, thereby facilitating a release of the sensor module 101 from carriage 104.

In Figure 5d, the user removes the device 100 from their skin, leaving an implanted and activated sensor module 101 in the user's skin 401. As the device 100 is removed, the collar 103 moves 503 relative to the body 107 to thereby surround the cannula 105 and protect the user from accidental injury. The collar 103 may move due to e.g. a spring force from spring 106, gravity and/or a combination of the two. In implementations, the collar 103 may return to its original position, for example due to being biased to the first position by the spring 106. An adhesive (e.g. an adhesive patch) may be provided on the user's skin to hold the sensor module 101.

In implementations, the (axial) spring force from spring 106 may be sufficient for spring 106 to function as an automatic cannula retraction mechanism. For example, the spring 106 may exert an upward force on body 107 and/or a corresponding downward force on collar 103 sufficient to lift the body 107 (and therefore the carriage 104) away from the user's skin 401 or otherwise bias the body and/or collar to the first position. Whether manually (e.g. by the user lifting the device) or automatically (e.g. by the user releasing the device), the movement of the body 107 (and therefore the carriage 104) relative to the user's skin 401 pulls or retracts the cannula 105 out of the epidermis while leaving the analyte sensor implanted in the skin 401. It is also envisaged that other automatic cannula retraction mechanisms may be provided, as will be appreciated by the skilled person.

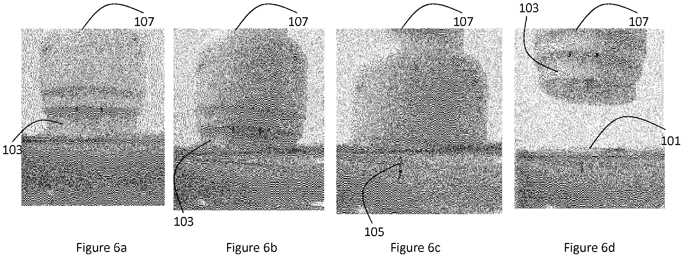

Figure 6a-d depict photographs of an example implementation of an inserter device according to the present disclosure. Figures 6a-d depict the inserter device securing a sensor module to a silicone model. The steps shown in Figures 6a-d correspond to Figures 5a-d respectively, and like reference numerals are provided for corresponding components.

Figures 7a-c illustratively show a forward end of the inserter device 100. Carriage 104 comprises grip jaws 701 for holding or securing the sensor module 101 within the carriage 104. Each of the grip jaws 701 comprises a protrusion or engagement surface configured to engage with a corresponding jaw recess or abutment surface 703 of the sensor module 101. While four grip jaws 701 are depicted, it will be appreciated that more generally the device 100 may comprise two or more grip jaws, and the number of grip jaws may be configured or selected based on e.g. a shape of the specific sensor module(s) intended for use with the inserter device.

Alternatively, it will be appreciated that the grip jaws 701 may engage with e.g. the surface of the sensor module 101 rather than a jaw recess 703, and/or that the sensor module may comprise protrusions in place or jaw recess 703, the protrusions configured to engage with corresponding recesses or slits of the carriage 104, such as recesses or slits provided on the grip jaw arms.

Figure 7a depicts the device 100 in a primed state prior to use. The primed state may correspond to the state of device 100 as shown in e.g. Figure 4a, such that the collar is in the first position. Collar 103 comprises collar jaw posts 702 configured to engage the grip jaws 701. The collar jaw posts 702 push or otherwise bias the grip jaws 701 radially inwards such that the grip jaws 701 firmly engage the jaw recesses 703 of the sensor module 101, to thereby securely hold the sensor module 101 within the carriage 104. In Figure 7a, the grip jaws 701 are in a sensor holding position.

Figure 7b depicts the device 100 in a delivered or delivery state, for securing the sensor module to a user's skin. The delivered state may correspond to the state of device 100 as shown in e.g. Figure 4b, such that the collar is in the second position. The collar 103 is retracted within the body 107, thereby releasing the grip jaws 701 from the collar jaw posts 702. The grip jaws 702 therefore relax to their original or free state, moving away from the jaw recesses 703 and loosening a grip on the sensor module 101. In Figure 7b, the grip jaws 701 are in a sensor release position.

Figure 7c depicts the device 100 in a released state, e.g. after securing the sensor module 100 to a user's skin. The released state may correspond to the state of device 100 as shown in e.g. Figure 4c. Grip jaws 701 may be deformable or elastically deformable, or otherwise connected to carriage 104 via spring hinged arms 701a. As such, a normal force exerted by the user's skin on the grip jaws 701 may further push the grip jaws 701 radially outwards and away from the jaw recesses 703, thereby fully releasing the sensor module 101 from the device 100. Sensor module may be secured to the user's skin via an adhesive patch (not shown) pre-applied to the user or otherwise attached to the sensor module or the device.

Alternatively, the grip jaws 701 and collar jaw posts 702 may be configured such that when the body 107 reaches an end of its stroke (i.e. when the collar 103 is in the second position and retracted into the body 107) the grip jaws 701 fully release the sensor module without e.g. deformation or other contact with the skin of the user. For example, the grip jaws 701 may be biased to a position for releasing the sensor module, and held in the sensor holding position by the collar jaw posts 702. As such, the disengagement of the grip jaws and collar jaw posts (e.g. resulting from the relative axial movement of the collar 103 and body 107 to the second position) may result in release of the sensor module. Further alternatively, grip jaws 701 in the sensor release position (e.g. as depicted in Figure 7b) may loosely grip the sensor module, such that an adhesive may secure the sensor module to the user's skin and remove the sensor module 101 from the carriage 104 as the user removes the device 100.

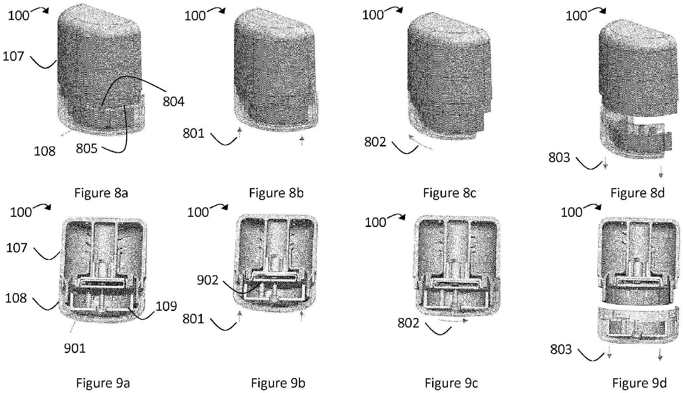

Figure 8a-d illustratively depict an example cap release process for inserter device 100. In Figure 8a, cap 108 is attached to a front or forward end of the body 107. The body 107 comprises a protrusion 804, while the cap 108 comprises a corresponding groove 805 configured to engage with the protrusion 804. The groove 805 comprises a first or locking section configured to secure the cap 108 to the body 107, a second or release section configured for inserting and removing the protrusion 804 from the groove 805, and a connection path between the locking and release sections.

As shown in Figure 8b, to remove the cap the user initially pushes 801 the cap 108 "inwards" (i.e. towards the body 107). In implementations, pushing the cap 108 in may activate a light in the cap 108 (e.g. by completing a circuit), thereby providing confirmation to the user that the cap 108 is correctly positioned. The body protrusion 804 correspondingly moves from the locking section of the groove 805 to the connection path.

As shown in Figure 8c, the user then twists 802 or rotates the cap 108 relative to the body 107 from a first (locked or secured) position to a second (release) position. The cap light may remain active while the cap 108 is twisted. The body protrusion 804 correspondingly moves along the connection path from the locking section to the release section.

Finally, in Figure 8d the user pulls off 803 the cap 108 to thereby remove the cap 108 from the body 107. The cap light may turn off when the cap 108 is removed.

Figures 9a-d illustratively depict a cross section view of inserter device 100 during an example cap release process. Figures 9a-d correspond to the respective steps shown in Figures 8a-d.

As shown in Figure 9a, cap 108 comprises a start-up ring or activator 109. The start-up ring 109 in turn comprises a mechanism for activating or turning-ON the sensor module 101. In the depicted implementation, the mechanism is switch on post 901, but it will be understood that other switch-ON mechanisms suitable for a particular sensor module may be used.

In Figure 9b, the user pushing 801 the cap 108 towards the body 107 thereby presses the start-up post 901 into an activation area 902 of the sensor module 101. The flexible activation area 902 may in turn push an activation switch on a control unit of the sensor module 101, thereby turning-ON the sensor module. The control unit may be e.g. a PCB.

In Figure 9c, the cap 108 is rotated 802. Start-up ring 109 may remain stationary relative to the body 107 due to grooves or guide rails provided within the collar 103. Corresponding protrusions on an outer wall of the start-up ring 109 may engage with the grooves of the collar 103, thereby preventing rotation of the start-up ring 109 as the user twists the cap 108.

Finally, in Figure 9d, the user removes the cap 108. Start-up ring 109 is retained within the cap 108. The sensor module 101 remains switched-ON, and the inserter device 100 is in a primed state ready for securing the sensor module to a user's skin.

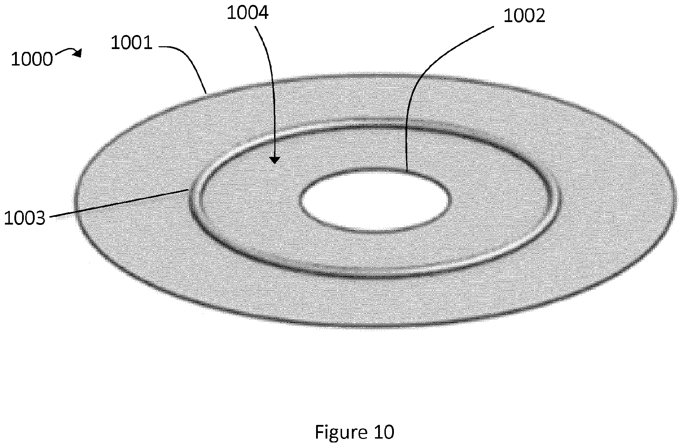

Figure 10 illustratively shows an example epidermal support patch 1000 suitable for use with the inserter device according to the present disclosure. The example patch 1000 is circular with an outer perimeter 1001 and an inner perimeter 1002 defining a central hole through which the cannula and analyte sensor of the sensor module are inserted into the skin.

The surface of the patch 1000 visible in Figure 10 may remain exposed after it is applied to the skin. The surface on the opposite side is provided with a biocompatible adhesive configured to secure the patch 1000 to the user's skin. The exposed surface may optionally define a raised portion 1003 at least partially surrounding a central area 1004 of the patch 1000. The raised portion 1003 has a ring like shape. It is envisaged the raised portion is integral with the patch, for example made of the same or similar material as the patch 1000, or alternatively made of a different material to the patch, for example a material that is harder than the patch 1000. The raised portion 1003, and accordingly the central area 1004 defined thereby, has a perimeter corresponding substantially to the perimeter of the collar 103 of the inserter device 100 i.e. the portion of the inserter device 100 that is in contact with the patch 1000 during use. Thus, the raised portion 1003 acts as a guide to position the inserter device 100 correctly and centrally on the patch to ensure correct placement thereof onto the patch. The patch 1000 may therefore be applied at any time prior to use to the patient's skin and the raised portion used to ensure correct placement of the inserter device at any time thereafter.

Alternatively, the patch may be pre-affixed to the sensor module or to the inserter device 100 itself, such that the process of fixing the sensor module to the user's skin affixes the sensor module to the use with the patch.

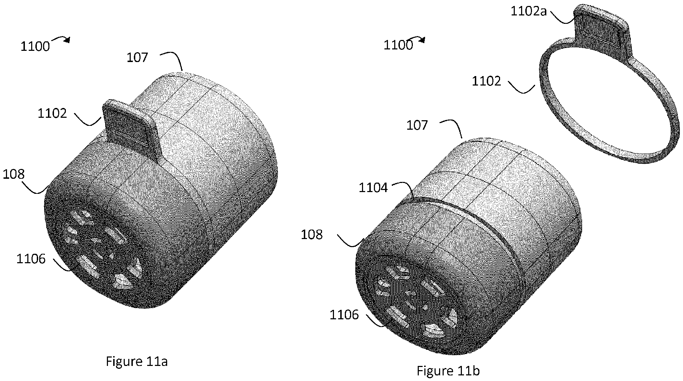

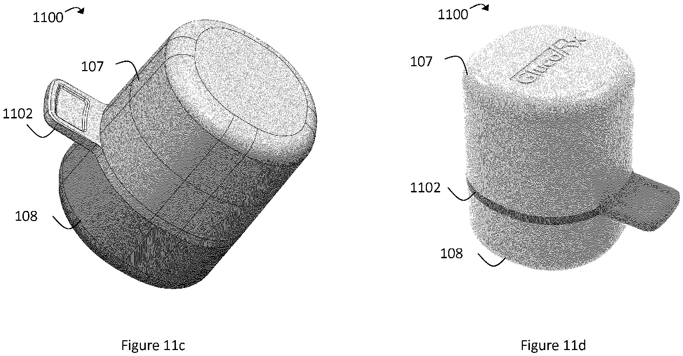

Figures 11a-d illustratively show views of a further applicator or inserter device 1100 according to the present disclosure. Several components of inserter device 1100 correspond to that of inserter device 100, and like reference numerals are used for these components. In particular, the internal components and structure of device 1100 may be identical to those of device 100.

Inserter device 1100 comprises a ring pull or seal ring 1102 positioned between the body 107 and cap 108 prior to use. The seal ring 1102 may be positioned in a groove or recess 1104 to thereby prevent or minimise accidental movement of the cap 108 "inwards" (i.e. towards the body 107). Advantageously, the seal ring 1102 may prevent or reduce accidental or unintentional activation of the sensor module and/or removal of the cap 108 from the body 107 under normal conditions (e.g. without damaging the device).

The seal ring 1102 may be formed from an elastically deformable material and/or from an elastomeric material, such that the ring 1102 can be stretched and deformed for removal from the device 1100, while still providing a secure and tight fit within groove 1104 prior to use of the device. The seal ring 1102 may comprise a tab 1102a or other member for gripping by the user, to aid in its removal from the device. After removing the seal ring 1102 (e.g. as shown in Figure 11b), the user may proceed to remove the cap 108 from the body 107 to activate the device for use, for example using the method steps shown in Figure 8 and 9 or by any other suitable method.

Also shown in Figures 11a and b are cap through holes or apertures 1106. The apertures 1106 may facilitate the insertion of a sterilising gas such as Ethylene Oxide into the device, to enhance the shelf life and safe use period of the inserter device. Corresponding through holes or apertures may also be provided within a start-up ring such as start-up ring 109. In implementations, the cap 108 may be rotatable to thereby misalign the apertures of the cap 108 and the apertures of the start-up ring after the insertion of the sterilising gas, e.g. to trap the gas within the body of the device. It will be understood that such apertures may optionally be provided in any or all implementations of the inserter device according to the present disclosure.

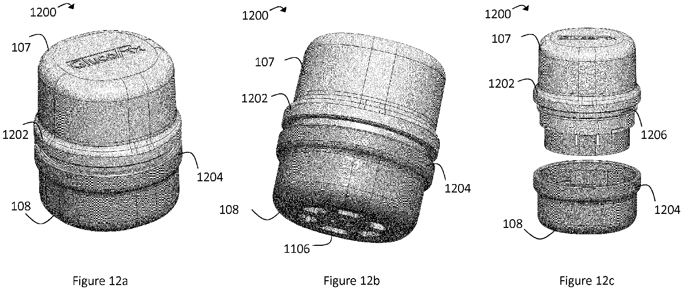

Figures 12a-c illustratively show views of another further applicator or inserter device 1200 according to the present disclosure. Several components of inserter device 1200 correspond to that of inserter device 100, and like reference numerals are used for these components. In particular, the internal components and structure of device 1200 may again be identical to those of device 100.

The body 107 and cap 108 of inserter device 1200 comprise corresponding flanges 1202 and 1204. A seal or gasket 1206 is provided on body 107 adjacent to flange 1202, such that when the cap 108 is attached to the body 107 the gasket 1206 is positioned between the flanges 1202 and 1204. Gasket 1206 may be formed from any suitable material, such as an elastomeric material.

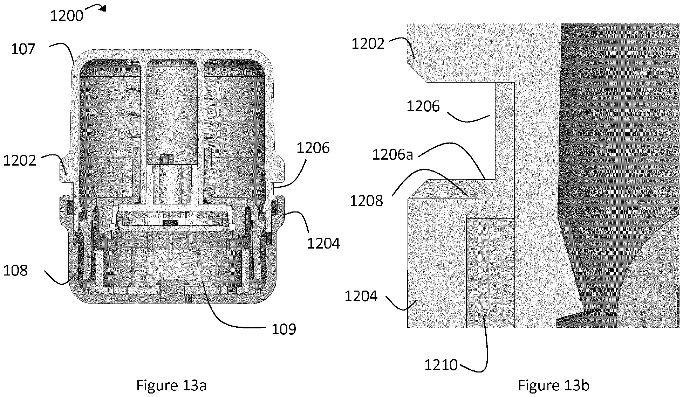

Figure 13a and b illustratively show cross section views of the device 1200 and gasket 1206. The gasket 1206 comprises a lip seal 1206a about the edge of the cap 108, to thereby positionally secure the cap 108 relative to the body 107. In the implementation shown, the lip seal 1206a comprises a groove or recess configured to receive a ridge, protrusion or other engagement surface 1208 on the inner surface of the cap flange 1204. The engagement of the engagement surface 1208 and the lip seal 1206a may increase a force required to move the cap 108 from its initial position relative to body 107.

After the disengagement of the engagement surface 1208 and the lip seal 1206a (e.g. due to the user pushing the cap "inwards" or towards the body 107), the lip seal 1206a may sit within a cavity 1210 between the flange 1204 and the body 107, such that it does not obstruct further relative movements of the cap 108 and the body 107 during a cap removal process. Advantageously therefore, the gasket may reduce or prevent accidental removal of the cap 108, by increasing an initial force required to begin the cap removal process.

It will be understood that a seal ring such as seal ring 1102 of device 1100 may be provided in addition to or instead of the flanges and gasket of inserter device 1200.

In implementations, the use of gasket 1206 may simplify the cap removal process relative to that shown in Figures 8 and 9. For example, the gasket 1206 may positionally secure the cap 108 relative to the body 107 prior to use of the inserter device, thereby requiring the user only to push the cap 108 "inwards" (towards the body 107) to activate the sensor module, and then remove the cap 108 by pulling the cap "outwards" or away from the body. However, it will be understood that the cap removal process of Figures 8 and 9 may also be used in conjunction with gasket 1206 and flanges 1202, 1204.

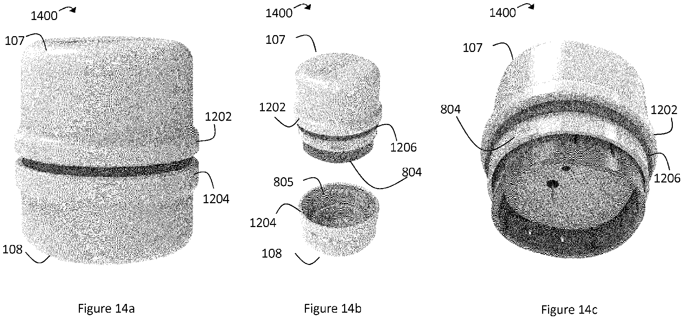

Figure 14a-c illustratively show views of an applicator or inserter device 1400 according to the present disclosure. Several components of inserter device 1400 correspond to that of inserter devices 1200, and like reference numerals are used for these components. In particular, the internal components and structure of device 1400 may be identical to those of device 1200, while the flanges 1202, 1204 and gasket 1206 may also function identically to the corresponding features described in respect to inserter device 1200.

Relative to device 1200, inserter device 1400 additionally comprises a protrusion 804 on an outer wall of body 107 and a groove 805 on an inner wall of cap 108, the groove 805 configured to engage with the protrusion 804. The protrusions 804 and 805 may be used to perform the cap removal steps depicted in Figures 8 and 9.

Other effective alternatives will occur to the skilled person. It will be understood that the invention is not limited to the described embodiments and encompasses modifications apparent to those skilled in the art lying within the scope of the claims appended hereto. Features described in relation to a single embodiment may be combined with or added to other embodiments describe herein.

It will be understood that relative positional terms such as "down" or "downward", "forward", "inward", etc. are made for illustrative purposes only in reference to the accompanying drawings, and are not intended to be limiting in nature.

For example, whilst the inserter device is described in the context of blood glucose analyte sensors, it is envisaged that the inserter device may be used with sensor modules comprising any implantable continuous analyte sensor, including for example, sensors for lactate, b-hydroxybutyrate, ethanol, cholesterol, and/or uric acid levels.

## Claims

1. An inserter device for securing a sensor module to the skin of a user, the inserter device configured, during use, to drive a pointed end of the sensor module at least partially into the skin of the user, the inserter device comprising:
a body housing a carriage for receiving the sensor module therein before use;
a collar radially surrounding the carriage and movable relative to the body in an axial direction from a first position to a second position, the collar comprising one or more engagement members to configured to engage one or more corresponding abutment surfaces on an inner surface of the body in the first position and thereby prevent relative movement of the collar and the body;
wherein the one or more engagement members and/or abutment surfaces are configured such that an axial force applied to the body causes the one or more engagement members and abutment surfaces to disengage from one another, thereby facilitating movement of the collar from the first position to the second position.

2. An inserter device according to claim 1, wherein the collar is configured to move in the axial direction relative to the body upon the application of the axial force that causes the one or more engagement members and the one or more abutment surfaces to disengage from one another.

3. An inserter device according to claim 1 or 2, wherein at least one of
(i) the one or more engagement members; and
(ii) the one or more abutment surfaces;
comprises a deformable section, wherein the axial force deforms the deformable section such that the one or more engagement members and the one or more abutment surfaces disengage from one another; and optionally wherein at least one of: the one or more engagement members comprise the deformable section; and wherein the one or more abutment members are configured such that the axial force causes the one or more abutment members to deform the deformable sections radially inwards; and the deformable sections are elastically deformable.

4. The inserter device of any preceding claim, wherein the carriage comprises a plurality of gripping members configured to secure the sensor module within the carriage, the gripping members configured to release the sensor module upon movement of the collar to the second position; and
optionally wherein the collar comprises a plurality of jaw posts, the jaw posts each configured to engage a respective one of the gripping members in the first position and exert a radially inward force on the gripping members; and further optionally wherein the jaw posts are configured such that movement of the collar to the second position causes the jaw posts to disengage the respective gripping members.

5. The inserter device of any preceding claim, wherein the inserter device comprises a force applicator, the force applicator configured to apply a second axial force from the body to the collar; and
optionally wherein the force applicator is a spring.

6. The inserter device of claim 5, wherein the force applicator biases the collar to the first position.

7. The inserter device of any preceding claim, wherein the carriage comprises a protrusion configured, during use, to guide the at least one pointed end of the sensor module to pierce the skin of the user when the collar is in the second position; and
optionally wherein the protrusion is a cannula.

8. The inserter device of claim 7, comprising an automatic cannula retraction mechanism; and
optionally wherein the force applicator is the automatic cannula retraction mechanism.

9. The inserter device of any preceding claim, further comprising a cap removably connected to the body, the cap configured to enclose a cavity of the body, the cavity for housing the sensor module; and
optionally wherein the cap comprises a sensor module activation member, wherein the cap is configured such that the process of removing the cap from the body causes the sensor module activation member to activate the sensor module housed within the body.

10. The inserter device according to claim 9, further comprising a seal ring removably positioned between the body and the cap, the seal ring configured to prevent a disconnection of the cap and the body; and
optionally wherein the seal ring comprises an elastically deformable or elastomeric material.

11. The inserter device of claim 9 or 10, wherein:
a first end of the cap comprises a first flange, the first end of the cap being axially proximate to the body;
the body comprises a second flange; and
the inserter device comprises a gasket positioned between the first flange and the second flange; and
optionally wherein the first flange comprises an engagement surface configured to engage with the gasket.

12. The inserter device of claim 11, wherein inserter device comprises a second cavity between the body and the first flange, the second cavity configured to receive the gasket.

13. The inserter device of any one of claims 9 to 12, wherein the cap further comprises one or more apertures for insertion of a gas into the cavity.

14. A kit of parts for securing a sensor module to the skin of a user, the kit of parts comprising:
an inserter device according to any preceding claim; and
a sensor module, the sensor module having a non-biocompatible adhesive on a surface thereof for securing the sensor module to the user;
the inserter device configured to, during use, move the sensor module into contact with the user to thereby secure the sensor module to user using said non-biocompatible adhesive.

15. The kit of parts of claim 14, comprising an epidermal support patch having a biocompatible adhesive on a surface thereof for securing the patch to the skin of a user; and
wherein securing the sensor module to the user comprises affixing the patch to the user and moving the sensor module into contact with the patch, to thereby secure the sensor module to the patch using said non-biocompatible adhesive.
